(19) Europäisches Patentamt
European Patent Office
Office européen des brevets

(11) **EP 3 223 748 B1**

(12) **FASCICULE DE BREVET EUROPEEN**

(45) Date de publication et mention de la délivrance du brevet:
**12.09.2018 Bulletin 2018/37**

(21) Numéro de dépôt: **15800824.3**

(22) Date de dépôt: **25.11.2015**

(51) Int Cl.:
***A61F 2/00*** ^(2006.01)

(86) Numéro de dépôt international:
**PCT/EP2015/077586**

(87) Numéro de publication internationale:
**WO 2016/083428 (02.06.2016 Gazette 2016/22)**

(54) **SYSTEME OCCLUSIF IMPLANTABLE**

IMPLANTIERBARES OKKLUSIONSSYSTEM

IMPLANTABLE OCCLUSION SYSTEM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **25.11.2014 FR 1461420**

(43) Date de publication de la demande:
**04.10.2017 Bulletin 2017/40**

(73) Titulaire: **Uromems**
**38000 Grenoble (FR)**

(72) Inventeur: **LAMRAOUI, Hamid**
**F-38000 Grenoble (FR)**

(74) Mandataire: **Regimbeau**
**87 rue de Sèze**
**69477 Lyon Cedex 06 (FR)**

(56) Documents cités:
| | |
|---|---|
| **EP-A1- 1 584 303** | **WO-A1-2014/118335** |
| **WO-A2-2009/056750** | **AU-A1- 2002 352 060** |
| **US-A- 4 222 377** | **US-A- 6 135 945** |

**Description**

**DOMAINE DE L'INVENTION**

La présente invention concerne un système occlusif implantable dans le corps humain ou animal.

**ARRIERE PLAN DE L'INVENTION**

Il est connu d'occlure un conduit anatomique au moyen d'un système occlusif implantable dans le corps d'un patient.

L'occlusion du conduit anatomique est assurée par une manchette gonflable remplie de fluide qui exerce une pression plus ou moins forte sur la partie à occlure en fonction du volume du fluide dans la manchette gonflable.

Par exemple, différents sphincters artificiels urinaires se basent sur ce principe pour exercer une pression sur l'urètre. Parmi les produits connus, on peut citer l'implant référencé AMS800 commercialisé par la société American Medical Systems ou bien l'implant référencé ZSI375 commercialisé par la société Zéphyr. On trouve le même principe dans d'autres types d'applications tels que les anneaux gastriques qui comportent une manchette gonflable placée autour de l'estomac.

La manchette gonflable remplie de fluide peut être réalisée sous différentes formes, par exemple entourant totalement ou en partie le conduit à occlure et peut être formée de différents matériaux biocompatibles, tels que du silicone implantable, du polyuréthane implantable, etc.

L'injection et l'aspiration de fluide dans la manchette gonflable nécessaire à l'occlusion de la partie anatomique peuvent être réalisées soit de manière manuelle et passive telle que pour les sphincters urinaires artificiels AMS800 et ZSI375, soit de manière automatique et active (à partir d'une source d'énergie électrique par exemple) pour des implants plus évolués.

Pour permettre une régulation de la pression exercée sur le conduit à occlure, la manchette gonflable est en liaison fluidique avec un réservoir de fluide couplé à un actionneur configuré pour injecter du fluide du réservoir vers la manchette (pour augmenter la pression exercée sur le conduit anatomique) ou de la manchette vers le réservoir (pour réduire la pression exercée sur le conduit anatomique). L'ensemble de la manchette gonflable, du réservoir et de la liaison fluidique entre eux forme un circuit fluidique.

Dans un tel système occlusif, il peut être nécessaire de mesurer la pression dans la manchette gonflable ou en un autre point du circuit fluidique, par exemple pour vérifier la pression lorsque l'actionneur est désactivé, ou encore pour contrôler la pression créée par ledit actionneur.

Le document EP 1 584 303 divulgue ainsi l'utilisation d'un capteur de pression implanté sur la manchette occlusive. Toutefois, une telle solution n'est pas réalisable industriellement car elle pose des problèmes d'intégration, d'encombrement, d'étanchéité et de biocompatibilité du capteur.

Il existe différents types de capteurs de pression.

Parmi les capteurs envisageables dans un système implantable, des capteurs de pression basés sur une membrane souple en contact avec le fluide pourraient être utilisés. Ces capteurs doivent néanmoins être biocompatibles, stables dans le temps, et il est nécessaire d'assurer une parfaite étanchéité du capteur pour éviter une infiltration de fluide ou d'humidité dans le capteur ou l'électronique associée.

Une solution à ce problème pourrait être l'utilisation d'un capteur de pression comprenant une membrane métallique souple assurant l'étanchéité du système. Cependant, un tel capteur a plusieurs inconvénients. D'une part, la membrane métallique du capteur étant fine, les procédés de fabrication peuvent être délicats. En effet, les contraintes mécaniques dues aux effets thermiques de la soudure sur la membrane peuvent avoir un effet sur la raideur de la membrane, ce qui peut induire des disparités significatives des propriétés mécaniques de la membrane. Par ailleurs, ce type de capteur est généralement scellé et rempli d'un fluide non compressible en contact avec un capteur de pression proprement dit. Le procédé d'assemblage des différentes parties du système (composé de plusieurs dizaines d'éléments) est donc délicat et coûteux. Enfin, lorsque le système est implanté, la fibrose entourant les différents éléments de l'implant peut induire un changement de raideur de la membrane et donc une dérive des mesures dans le temps.

Un autre problème à résoudre est de pouvoir appliquer une pression définie et précise sur le conduit anatomique en consommant un minimum d'énergie.

Une solution simple serait d'utiliser un système basé sur la pression d'occlusion mesurée. Parmi les moyens de mesure de la pression d'occlusion, on peut citer des systèmes qui mesurent directement la pression dans le circuit fluidique via un capteur adapté, ou bien qui mesurent la pression indirectement par exemple à partir du courant consommé par l'actionneur comme décrit dans le document US 8,585,580.

Cependant, il a été démontré par des tests in vivo [1] que la pression dans le circuit fluidique varie fortement et en permanence lors de l'occlusion. Dans le cas d'un système basé sur une régulation en pression, cela a pour effet de solliciter en quasi-permanence l'actionneur pour stabiliser la pression à une consigne donnée, avec pour conséquence d'induire une consommation électrique excessive du système.

D'autres principes ont été proposés. Par exemple, le document US 8,585,580 propose un système qui transfère un fluide à la manchette gonflable jusqu'à ce que la pression mesurée dépasse un seuil défini. Cette solution a pour inconvénient d'être peu précise sur la pression d'occlusion appliquée. En effet, lors de la phase d'occlusion, la pression peut augmenter fortement puis diminuer en raison de la relaxation des tissus et du dispositif d'occlusion. Le fluide apporté au dispositif d'occlusion en contact avec le conduit anatomique à occlure n'est donc généralement pas suffisant pour générer la pression désirée.

Par ailleurs, l'encombrement d'un tel capteur pose également un problème, dans la mesure où le système implantable doit être de dimensions aussi réduites que possibles et que ledit système comprend en outre un dispositif de transfert de fluide dont le volume peut être conséquent et une pile qui représente également une grande partie du volume du système implantable. L'intégration d'un tel capteur dans ce système peut être difficile en raison de l'encombrement dudit capteur. De plus, ce type de capteur devant être à la fois en contact avec l'extérieur, pour la mesure de pression, et avec l'intérieur, pour la communication avec le module électronique, il est nécessaire de mettre en oeuvre un processus de fabrication fiable et hermétique, tel que la soudure laser, qui peut être contraignant en phase de production.

## BREVE DESCRIPTION DE L'INVENTION

Un but de l'invention est de concevoir un système occlusif implantable qui permette de s'affranchir des inconvénients des systèmes existants. En particulier, ce système doit permettre de mesurer la pression dans la manchette de manière fiable tout en étant biocompatible, étanche et en minimisant l'encombrement du système implantable et la consommation d'énergie nécessaire à la régulation de cette pression. De manière préférée, ledit système devrait également permettre de contrôler la pression dans la manchette.

A cet effet, il est proposé un système occlusif implantable dans un corps humain ou animal, comprenant :

- un circuit fluidique comprenant :

  • une manchette occlusive gonflable contenant un volume variable d'un fluide, destinée à entourer au moins une partie d'un conduit naturel à occlure,
  • un réservoir à volume variable rempli d'un fluide, ledit réservoir comprenant une partie fixe et une partie mobile,
  • une liaison fluidique entre le réservoir et la manchette occlusive,

- un actionneur couplé mécaniquement à la partie mobile du réservoir de sorte à déplacer linéairement ladite partie mobile par rapport à la partie fixe pour ajuster le volume du réservoir,

l'actionneur et le réservoir à volume variable étant agencés dans un boîtier étanche contenant un gaz.

Conformément à l'invention, ledit système comprend en outre :

- un capteur agencé dans le boîtier, lié mécaniquement à l'actionneur et/ou à la partie mobile du réservoir, agencé de sorte à mesurer une force de traction et/ou de compression dans la direction de déplacement de la partie mobile du réservoir, ladite force mesurée résultant au moins de :

  • la force notée $F_{occl}$ exercée sur la partie mobile du réservoir à volume variable liée à la pression dans le circuit fluidique, et
  • la force notée $F_{boîtier}$ exercée sur la partie mobile du réservoir à volume variable liée à la pression dans le boîtier,

- un dispositif de mesure de la pression de fluide dans le circuit fluidique comprenant une unité de traitement configurée pour déterminer ladite pression de fluide à partir d'un calcul prenant en compte au moins la mesure de force dudit capteur, la surface de pression effective de la partie mobile du réservoir, et la force $F_{boîtier}$ exercée sur la partie mobile du réservoir à volume variable liée à la pression de gaz dans le boîtier.

Ce système présente les avantages suivants. D'une part, le fait que le capteur soit agencé dans le boîtier permet de mesurer la pression sans aucun élément déporté (externe au boîtier) et évite donc la mise en oeuvre de connexions complexes sortant du boîtier et dont l'étanchéité doit être assurée. En outre, la biocompatibilité est assurée par le boîtier et ne génère donc aucune contrainte spécifique en termes de conception du capteur. Enfin, le capteur étant intégré au système de pressurisation de la manchette, l'encombrement requis est minimisé.

Selon une forme d'exécution préférée, le système comprend en outre un dispositif de contrôle de la pression de fluide dans le circuit fluidique par le volume du réservoir, comprenant :

- une mémoire dans laquelle est enregistrée une relation entre la pression dans le circuit fluidique et le volume dudit réservoir,
- une unité de traitement configurée pour :

  - recevoir une consigne de pression de fluide dans le circuit fluidique,
  - à partir de la relation enregistrée dans la mémoire entre la pression dans le réservoir et le volume du réservoir, déterminer le volume du réservoir permettant d'atteindre la pression de consigne,
  - le cas échéant, commander l'actionneur pour déplacer la partie mobile du réservoir dans la position définissant ledit volume déterminé,

- une unité de calibration configurée pour :

  (a) lorsque le patient est dans une situation déterminée, commander l'actionneur pour déplacer la partie mobile du réservoir dans une pluralité de positions déterminées, chaque position définissant un volume déterminé du réservoir,
  (b) pour chacune desdites positions :

  - mesurer la pression de fluide dans le circuit fluidique par ledit dispositif de mesure de la pression de fluide dans le circuit fluidique,
  - mettre à jour la mémoire en enregistrant ladite pression de fluide mesurée dans le circuit fluidique pour le volume respectif du réservoir.

Selon un mode de réalisation, le capteur est apte à mesurer des forces de traction et des forces de compression dans la direction de déplacement de la partie mobile du réservoir.

Selon un autre mode de réalisation, le capteur est apte à mesurer uniquement des forces de compression dans la direction de déplacement de la partie mobile du réservoir ; dans ce cas, le système comprend en outre un dispositif de précontrainte agencé de sorte à exercer une précontrainte de compression déterminée sur ledit capteur.

Dans ce cas, l'unité de traitement est configurée pour prendre en compte ladite précontrainte pour la détermination de la pression de fluide dans le circuit fluidique.

Le dispositif de précontrainte comprend avantageusement au moins un ressort de compression, un ressort de traction et/ou un plot en élastomère.

Selon un mode de réalisation, la partie mobile du réservoir à volume variable comprend un système d'entrainement couplé à une paroi mobile et un soufflet déformable s'étendant et se comprimant en fonction de la position de ladite paroi mobile.

Dans ce cas, l'unité de traitement est avantageusement configurée pour prendre en compte la raideur dudit soufflet pour la détermination de la pression de fluide dans le circuit fluidique.

Dans une forme d'exécution avantageuse, le système d'entraînement comprend une vis solidaire de la paroi mobile et une noix couplée à la vis et mobile en rotation sur une butée à billes à double effet autour de l'axe de la vis sous l'effet d'une action d'entraînement par l'actionneur, la noix étant couplée à la vis de sorte qu'une rotation de la noix entraîne la vis uniquement en translation dans la direction de déplacement de la partie mobile ; par ailleurs, le capteur est agencé dans ladite butée à billes de sorte à mesurer au moins une force de traction et une force de compression dans la direction de déplacement de la partie mobile du réservoir.

Selon un autre mode de réalisation, la partie mobile du réservoir à volume variable comprend un système d'entrainement couplé à une membrane roulante.

Selon un autre mode de réalisation, le réservoir à volume variable comprend un cylindre formant la partie fixe du réservoir et un piston coulissant dans ledit cylindre, formant la partie mobile du réservoir.

De manière avantageuse, l'actionneur est choisi parmi les actionneurs piézoélectriques, les actionneurs électromagnétiques, les polymères électro-actifs et les alliages à mémoire de forme.

Selon une forme d'exécution de l'invention, le système comprend en outre un capteur de pression de gaz agencé dans le boîtier pour la mesure de la pression de gaz dans le boîtier, l'unité de traitement étant configurée pour prendre en compte ladite pression de gaz mesurée dans la détermination de la force $F_{boîtier}$.

De manière avantageuse, une paroi du réservoir à volume variable est constituée par une paroi du boîtier, ladite paroi comprenant un port de ponction perforable.

De manière avantageuse, le système comprend en outre un dispositif de réduction des contraintes dans le circuit fluidique lorsque lesdites contraintes dépassent un seuil déterminé.

Selon un mode de réalisation préféré, le système comprend en outre un accéléromètre, l'unité de traitement étant configurée pour, à partir des données de mesure de l'accéléromètre, déterminer si le patient est dans une situation déterminée.

De manière particulièrement avantageuse, le dispositif de mesure de la pression est configuré pour mesurer la pression de fluide lors de l'ajustement du volume du réservoir et pour vérifier la correspondance entre ladite valeur mesurée et une valeur attendue.

Selon une application préférée de l'invention, le système est un sphincter urinaire artificiel.

Selon un mode de réalisation, l'unité de traitement est configurée pour calculer une pression relative de fluide dans le circuit fluidique, ladite pression relative étant égale à la différence entre la pression de fluide déterminée dans le circuit fluidique et la pression atmosphérique s'exerçant sur le patient.

Selon une forme d'exécution, le système comprend en outre un dispositif destiné à être placé à l'extérieur du corps du patient et comprenant un capteur barométrique adapté pour mesurer la pression atmosphérique s'exerçant sur le patient, ledit dispositif étant capable de communiquer ladite mesure de pression à l'unité de traitement pour le calcul de ladite pression relative du fluide dans le circuit fluidique.

De manière alternative, l'unité de traitement est configurée pour déterminer la pression atmosphérique s'exerçant sur le patient à partir de la pression dans le circuit de fluide et d'une pression relative connue dans une configuration d'actionnement donnée de la manchette.

## BREVE DESCRIPTION DES DESSINS

D'autres caractéristiques et avantages de l'invention ressortiront de la description détaillée qui va suivre, en référence aux dessins annexés sur lesquels :

- la figure 1 est une vue d'ensemble du système occlusif implantable,
- la figure 2 est une vue en coupe de l'intérieur du boîtier d'un système occlusif implantable selon un premier mode de réalisation de l'invention,
- la figure 3 est une vue en coupe du boîtier d'un système occlusif implantable selon un deuxième mode de réalisation de l'invention,
- la figure 4 est une vue en coupe du boîtier d'un système occlusif implantable selon un troisième mode de réalisation de l'invention,
- la figure 5 est une vue en coupe de l'intérieur du boîtier d'un système occlusif implantable selon un quatrième mode de réalisation de l'invention,
- la figure 6 est un schéma présentant les différentes forces susceptibles d'être mesurées par le capteur de force de compression ou de traction, en présence d'une précontrainte,
- la figure 7 est un schéma présentant les différentes forces susceptibles d'être mesurées par le capteur de force de compression et de traction, en l'absence de précontrainte,
- la figure 8 est un graphe présentant la variation de la pression dans le circuit fluidique en fonction du volume injecté dans la manchette occlusive,
- la figure 9 est un graphe présentant la variation de la pression dans le circuit fluidique en fonction du temps pour différents volumes de fluide injectés dans la manchette occlusive lors de la procédure de calibration,
- la figure 10 est une vue en coupe du boîtier d'un système occlusif implantable selon un cinquième mode de réalisation de l'invention,
- la figure 11 est une vue en coupe du capteur de force et de l'actionneur du système de la figure 10, avec une représentation des forces mesurées.

## DESCRIPTION DETAILLEE DE MODES DE REALISATION DE L'INVENTION

Présentation générale du système occlusif implantable

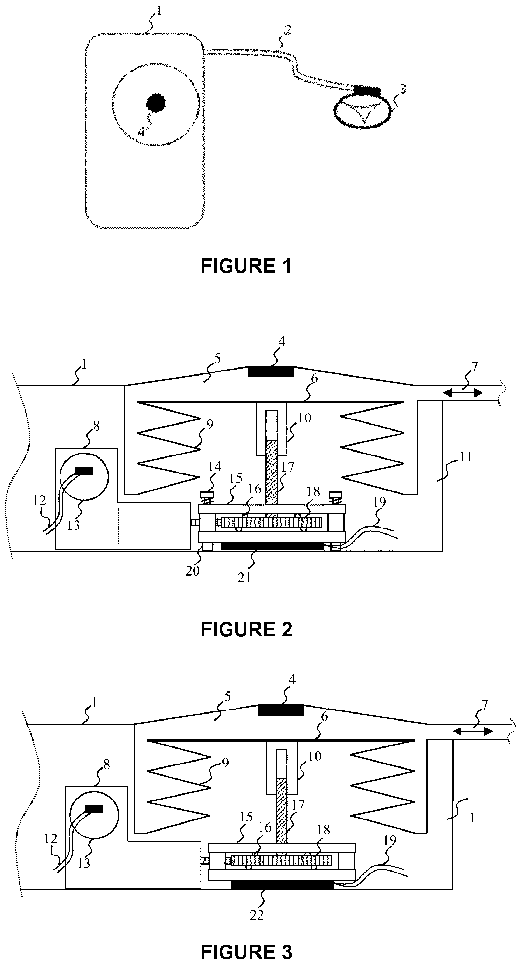

En référence à la figure 1, le système occlusif comprend une manchette occlusive 3 gonflable contenant un volume variable d'un fluide, destinée à entourer au moins une partie d'un conduit naturel (non illustré) à occlure, et un réservoir 5 à volume variable (représenté sur les figures 2 à 5) rempli d'un fluide.

La manchette occlusive peut être réalisée en élastomère biocompatible (cf. par exemple les documents US4222377, CA1248303 ou US4408597).

Ledit réservoir comprend une partie fixe et une partie mobile, le déplacement de la partie mobile faisant varier le volume du réservoir.

A cet effet, le système occlusif comprend un actionneur couplé mécaniquement à la partie mobile du réservoir de sorte à déplacer linéairement ladite partie mobile par rapport à la partie fixe pour ajuster le volume du réservoir. L'actionneur peut notamment comprendre un moteur électromagnétique et un réducteur. L'actionneur est commandé par un dispositif de contrôle de la pression de la manchette qui sera décrit en détail plus bas.

Pour chaque volume du réservoir, la partie mobile présente une surface de pression effective connue, qui peut être constante ou variable suivant les modes de réalisation.

Le système occlusif comprend en outre une liaison fluidique 2 (typiquement une tubulure) entre le réservoir 5 et la manchette occlusive 3.

Ainsi, une variation de volume du réservoir 5 entraîne un ajout ou un retrait de fluide dans la manchette 3, faisant ainsi augmenter ou diminuer la compression exercée sur le conduit entouré par la manchette.

L'ensemble formé du réservoir à volume variable, de la manchette occlusive et de la liaison fluidique est appelé circuit fluidique dans la suite de la description.

En plus du dispositif de contrôle de la pression de la manchette occlusive, le système implantable comporte un ou plusieurs modules électroniques permettant de réaliser toutes les fonctions nécessaires. Il comporte en outre une source d'énergie rechargeable ou non permettant d'alimenter le système. Dans une configuration particulière la source d'énergie est à l'extérieur du corps humain et transmet l'énergie sans fil au système implantable.

Le réservoir à volume variable, l'actionneur ainsi que le ou lesdits modules électroniques et, le cas échéant, la source d'énergie, sont agencés dans un boîtier 1 destiné à être implanté dans le corps du patient. Le boîtier 1 contient un gaz, par exemple de l'air. Ledit boîtier doit être étanche pour éviter tout transfert de fluide ou de gaz de ou vers le milieu intracorporel. Le boîtier est en un matériau biocompatible et peut par exemple être réalisé en titane implantable et scellé par soudure laser. Un contrôle de l'étanchéité peut notamment être réalisé à l'hélium (par exemple, taux de fuite inférieur à $10^{-9}$ mbar.l/s d'hélium) pour s'assurer de l'étanchéité totale du boîtier pendant la période pour laquelle le dispositif est implanté.

Selon un mode de réalisation particulier, le boîtier peut contenir un capteur de pression de gaz, dont la fonction sera décrite plus bas.

De manière avantageuse, le boîtier 1 comprend, dans une paroi délimitant le réservoir à volume variable, un port de ponction 4 perforable par une aiguille et apte à se refermer de manière étanche après le retrait de l'aiguille, permettant d'injecter ou de retirer du fluide du réservoir.

Le boîtier renferme également un capteur en liaison mécanique avec l'actionneur et/ou la paroi mobile du réservoir à volume variable pouvant mesurer une force de compression et/ou de traction dans la direction de déplacement de la partie mobile du réservoir.

Parmi les capteurs adaptés pour cet usage, on peut citer par exemple :

- un capteur basé sur une ou plusieurs jauges de contrainte (de telles jauges permettent de mesurer des forces de traction et de compression) ;
- un ou plusieurs capteurs de type FSR™ (acronyme du terme anglo-saxon « Force Sensing Resistor ») commercialisés par la société Interlink Electronics, mesurant des forces de compression) ; et/ou
- un ou plusieurs capteurs de pression couplés à un mécanisme permettant de mesurer une force. Par exemple, on peut citer un capteur de pression hydraulique combiné à une poche remplie de fluide et agencée de manière à mesurer une pression sur une surface prédéterminée, permettant ainsi d'en déduire la force appliquée à la surface de mesure.

Le système peut comprendre en outre un dispositif permettant de mesurer la pression atmosphérique dans l'environnement du patient. Un capteur barométrique capable de mesurer la pression atmosphérique courante exercée sur le corps du patient peut par exemple être agencé dans un dispositif externe porté par le patient. Cette mesure peut être transmise par liaison sans fil au dispositif de contrôle agencé dans le boîtier implantable.

Réservoir à volume variable

Selon un mode de réalisation préféré, le réservoir à volume variable comprend un soufflet assemblé dans le boîtier, le soufflet et le boîtier étant par exemple réalisés en titane implantable. Le réservoir à volume variable est alors constitué du soufflet (faisant office de partie mobile), d'une paroi du boîtier et d'un capot faisant office, avec ladite paroi du boîtier, de partie fixe. Le réservoir comprend en outre un orifice permettant de transférer le fluide de et vers l'extérieur du réservoir.

L'utilisation de soufflet métallique pour réaliser la fonction de réservoir à volume variable est connue de l'homme du métier (cf. document US4581018 par exemple). Un tel soufflet est par exemple commercialisé par les sociétés Servometer et Witzenmann.

Le soufflet présente l'avantage d'assurer une étanchéité totale de l'implant tout en autorisant le mouvement de la paroi mobile. Sa surface de pression effective peut être considérée constante sur toute la plage de course du soufflet.

Il faut néanmoins prendre en compte dans la conception du dispositif la raideur mécanique du soufflet qui peut avoir une influence sur le comportement du dispositif (impact sur le rendement énergétique, direction des forces, etc.). La manière dont ce paramètre est pris en compte sera décrite en détail plus bas.

Cependant, la présente invention n'est pas limitée à l'utilisation d'un soufflet pour former le réservoir à volume variable. Ainsi, l'homme du métier pourra mettre en oeuvre pour réaliser le réservoir à volume variable un piston ou une membrane roulante, qui sont considérés comme ne présentant pas de raideur mécanique. Dans ce cas, contrairement au cas du soufflet, la raideur sera considérée nulle ou négligeable dans le calcul de la pression.

La partie mobile du réservoir présente une surface de pression effective qui peut être constante ou variable selon la forme d'exécution du réservoir.

Dans le cas d'un soufflet, la surface de pression effective est considérée constante et est donnée par le fabricant. Pour une membrane roulante, la surface de pression effective varie en fonction la position de la membrane roulante et est donnée par le fabricant pour différentes valeurs de course,

Dans le cas d'un piston coulissant sans frottement dans un cylindre, la surface de pression effective est égale à la surface frontale du piston.

Actionneur

L'actionneur peut être choisi parmi tout système électromécanique permettant de transformer une énergie électrique en un mouvement mécanique avec la puissance requise pour permettre le déplacement à une force et à une vitesse requise de la partie mobile du réservoir à volume variable. On peut citer pour exemple, parmi les actionneurs connus de l'homme du métier, les actionneurs piézo-électriques, les moteurs électromagnétiques avec ou sans balais (dans le cas d'un moteur sans balais, celui-ci peut être constitué de 2 pôles ou de 4 pôles) couplés ou non à un réducteur, les polymères électro-actifs ou bien les alliages à mémoire de forme.

La figure 2 illustre un mode de réalisation de l'invention et représente une coupe d'une partie de l'intérieur du boîtier 1.

Le réservoir à volume variable 5 comprend une partie mobile qui, dans ce mode de réalisation, est un soufflet 9.

Le soufflet présente une flasque 6 couplée à une vis d'entraînement 17 par l'intermédiaire d'une noix 10 solidaire de la flasque 6 et présentant un taraudage coopérant avec le filetage de la vis 17.

Le réservoir est délimité par une partie de la paroi du boîtier 1 et le soufflet 9.

La paroi du boîtier 1 comprend par ailleurs un port de ponction 4 qui est perforable par une aiguille en vue d'ajouter ou de retirer du fluide du réservoir.

Le réservoir 5 comprend en outre une connexion 7 pour une tubulure assurant la liaison fluidique avec la manchette occlusive (non représentée).

Le boîtier 1 contient en outre un actionneur comprenant un moteur 13 couplé à un réducteur 8. Un connecteur 9 permet d'alimenter le moteur 13 lorsque le dispositif de contrôle émet un ordre de fonctionnement du moteur dans un sens ou dans l'autre selon qu'une augmentation ou une diminution du volume du réservoir est requise.

Le réducteur est couplé à une roue dentée 18 qui est elle-même couplée à la vis d'entraînement 17, de sorte à transmettre le couple et la rotation de l'axe du moteur 13 à la vis d'entraînement 17. La rotation de la vis 17 entraîne alors la noix 10 en translation, ce qui a pour effet de déplacer la flasque 6 en translation dans une direction parallèle à l'axe de la vis 17, le sens de déplacement de la flasque 6 dépendant du sens de rotation du moteur 13.

Le volume intérieur 11 du boîtier entourant le réservoir 5 est rempli d'un gaz.

La variation de volume du réservoir 5 a donc pour effet de faire varier le volume intérieur 11 et par conséquent la pression du gaz dans ledit volume 11.

Un capteur de pression de gaz (non illustré) peut éventuellement être agencé dans le volume 11 pour mesurer la pression dans ce volume.

La roue dentée 18 est logée dans un bloc 15 par l'intermédiaire de roulements à billes 16 qui permettent sa rotation dans le bloc 15. La roue dentée 18 étant solidaire de la vis d'entraînement 17, le bloc 15 est entraîné en translation dans le boîtier par la vis 17. Des plots 20 s'étendant à partir de la paroi du boîtier 1 parallèlement à l'axe de rotation de la vis 17 permettent de guider la translation du bloc 15.

Un capteur de force est fixé sur la paroi du boîtier en vis-à-vis de la face du bloc 15 opposée au réservoir 5. Le repère 19 désigne un connecteur permettant la transmission des données de mesure du capteur vers le dispositif de mesure et de contrôle de la pression.

Dans ce mode de réalisation, le capteur de force 21 ne mesure que des forces de compression.

Pour permettre de mesurer néanmoins des forces de traction, le système comprend un dispositif de précontrainte 14 qui exerce une force de compression déterminée sur le capteur de force. Ce dispositif de précontrainte crée ainsi un « offset » sur le capteur de force, ce qui permet de mesurer à la fois des forces de traction et des forces de compression.

Ledit dispositif de précontrainte peut comprendre une ou plusieurs vis de réglage coopérant avec les plots de guidage 20 et, interposé(s) entre la tête d'une vis de réglage respective et le bloc 15, un ressort de compression, un ressort d'extension et/ou un plot en élastomère.

Comme on le voit, l'intégration du capteur de force au système de pressurisation de la manchette est particulièrement compacte et ne pénalise donc pas l'encombrement du système implantable.

La figure 3 illustre un autre mode de réalisation de l'invention et représente une coupe d'une partie de l'intérieur du boîtier 1.

Les éléments portant les mêmes signes de référence que sur la figure 2 remplissent la même fonction et ne seront donc pas décrits à nouveau.

Dans ce mode de réalisation, le capteur de force 22 peut par exemple être basé sur une mesure par jauge(s) de contrainte. Le capteur 22 est fixé sur le bloc 15 et sur la paroi du boîtier en vis-à-vis dudit bloc, du côté opposé au réservoir. Le capteur de force 22 est apte à mesurer à la fois des forces de compression et de traction. Le dispositif de précontrainte du mode de réalisation de la figure 2 n'est pas nécessaire dans ce cas.

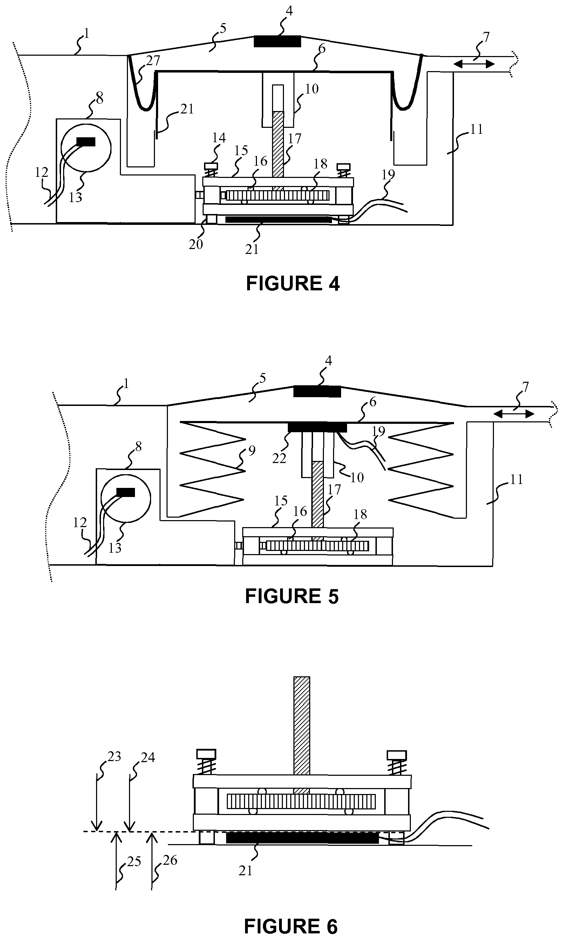

La figure 4 illustre un mode de réalisation de l'invention et représente une coupe d'une partie de l'intérieur du boîtier 1.

Dans ce mode de réalisation, la partie mobile du réservoir 5 n'est pas un soufflet mais une membrane roulante 27. Comme sur la figure 2, le capteur de force 21 est ne mesure que des forces de compression et est donc associé à un dispositif de précontrainte 14.

La figure 5 illustre un autre mode de réalisation de l'invention et représente une coupe d'une partie de l'intérieur du boîtier 1.

Les éléments portant les mêmes signes de référence que sur la figure 2 remplissent la même fonction et ne seront donc pas décrits à nouveau.

Dans ce mode de réalisation, le capteur de force 22 peut par exemple être basé sur une mesure par jauge(s) de contrainte. Il peut être intégré entre la noix taraudée 10 et la flasque 6. La noix taraudée 10 et le capteur de force 22 peuvent également constituer un ensemble unique et complet intégrant la ou les jauges de contrainte et une partie taraudée, remplissant ainsi la fonction de mesure de force et d'entrainement par la vis d'entrainement 17. Le capteur de force 22 est apte à mesurer à la fois des forces de compression et de traction. Le dispositif de précontrainte du mode de réalisation de la figure 2 n'est pas nécessaire dans ce cas.

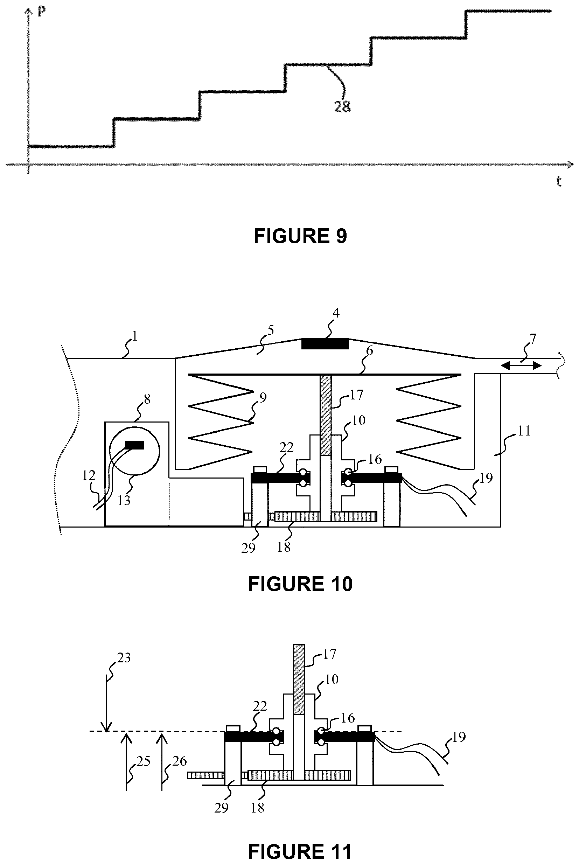

La figure 10 illustre un autre mode de réalisation de l'invention et représente une coupe d'une partie de l'intérieur du boîtier 1.

Les éléments portant les mêmes signes de référence que sur la figure 2 remplissent la même fonction et ne seront donc pas décrits à nouveau.

Dans ce mode de réalisation, la vis 17 est solidaire de la flasque mobile 6 du soufflet 9 et ne tourne pas. Le mouvement linéaire de la partie mobile supérieure 6 du soufflet 9 s'effectue grâce à la rotation de la noix 10 qui entraîne linéairement la vis 17. La noix 10 est solidaire de la roue 18 qui est entraînée par le réducteur 8. La noix 10 est agencée de telle sorte que seule une rotation autour de l'axe de la vis 17 est permise. Pour cela, une butée à bille à double effet est agencée sur le capteur de force 22, à l'aide des billes 16 qui permettent à la noix 10 de tourner et de supporter les forces subies suivant l'axe de la vis 17. Les forces exercées sur la noix 10 sont identiques à celle exercées dans les autres configurations.

La figure 11 représente les principales forces subies et mesurées par le capteur de force 22 dans le but d'en déduire la pression dans le circuit fluidique. Pour la mesure de force dans les deux directions suivant l'axe de la vis 17, le capteur de force 22 est intégré dans la butée à billes 16 de sorte à pouvoir mesurer des forces dans les deux directions. Les supports 29 permettent de maintenir le capteur de force 21. Dans ce mode de réalisation, les forces 23, 25 et 26 sont mesurées par le capteur de force au niveau de la butée à billes 16 intégrée dans la noix d'entraînement 10. Le capteur de force peut être dans ce cas un capteur comprenant des jauges de contrainte placées de sorte à mesurer des forces dans les deux directions suivants l'axe de la vis 17 solidaire de la flasque 6 du soufflet 9.

Le mode de réalisation illustré sur les figures 10 et 11 a l'avantage de permettre un gain d'espace et permet d'obtenir une course du soufflet plus importante pour les mêmes dimensions de l'implant, par rapport aux autres modes de réalisation. L'intégration mécanique est par ailleurs plus simple.

Détermination de la pression dans le circuit fluidique du système occlusif

La pression dans le circuit fluidique est déterminée indirectement. Comme il a été indiqué en préambule, l'intégration d'un capteur de pression sur l'une des parois du réservoir à volume variable ou sur l'une des parties du circuit fluidique serait contraignante et présenterait plusieurs inconvénients.

A la place d'un tel capteur, la présente invention utilise la partie mobile du réservoir à volume variable pour mesurer indirectement la pression dans le circuit fluidique du système occlusif.

Le système implantable comprend donc un dispositif de mesure de la pression de fluide dans le circuit fluidique, qui comprend ledit capteur de force et une unité de traitement (par exemple un microprocesseur) couplée audit capteur. L'unité de traitement prend en compte les mesures de force acquises par ledit capteur, ainsi que d'autres paramètres mécaniques, physiques et dimensionnels du système, pour déterminer la pression de fluide dans le circuit fluidique.

Afin de mesurer une pression relative dans le circuit fluidique, un capteur (par exemple, un capteur barométrique) permettant de mesurer la pression atmosphérique courante peut être agencé dans un dispositif externe porté par le patient afin de prendre en considération les variations de pression atmosphérique liées à l'altitude et/ou aux conditions météorologiques.

Un autre mode de réalisation peut être basé sur la mesure de pression atmosphérique courante directement à partir du circuit fluidique lorsque l'on a l'assurance dans des conditions déterminées d'avoir une pression dans le circuit fluidique égale à une pression relative connue (par exemple, pression relative nulle pour une position particulière de la partie mobile du réservoir).

La mesure de la pression atmosphérique est alors prise en compte dans les deux modes de réalisation décrits ci-dessous pour en déduire une pression relative à partir d'une mesure de pression absolue comme décrit ci-dessous.

La description ci-dessous se base sur un capteur de force ne mesurant que la compression (pas de traction). Le même principe peut être appliqué pour des capteurs de forces mesurant des compressions et des tractions. Dans ce cas, on s'affranchit du système de précontrainte permettant de créer un « offset » sur la force pour mesurer à la fois des forces de compression et de traction.

La figure 6 illustre les différentes forces mesurées par le capteur de force, dans le cadre du mode de réalisation de la figure 2 :

- le repère 23 désigne la force relative à la pression dans le circuit fluidique ;
- le repère 24 désigne la force de précontrainte ;
- le repère 25 désigne la force liée à la raideur du soufflet ;
- le repère 26 désigne la force liée à la pression de gaz dans le boîtier.

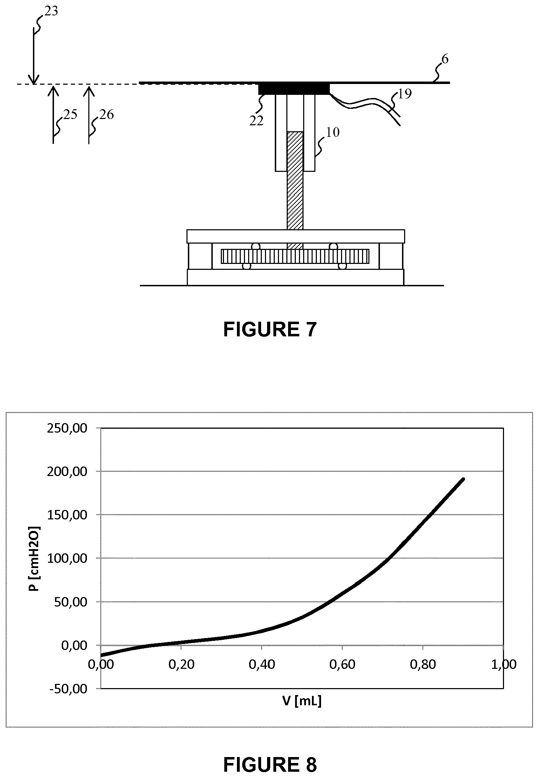

La figure 7 illustre les différentes forces mesurées par le capteur de force placé au niveau de la noix taraudée 10 et de la paroi mobile 6, dans le cadre du mode de réalisation de la figure 5 :

- le repère 23 désigne la force relative à la pression dans le circuit fluidique ;
- le repère 25 désigne la force liée à la raideur du soufflet ;
- le repère 26 désigne la force liée à la pression de gaz dans le boîtier.

Pour déduire la pression P1 dans le circuit fluidique, les paramètres pris en compte sont les suivants :

Seff : la surface de pression effective de la partie mobile du réservoir à volume variable (comme indiqué plus haut, cette surface peut être fixe ou variable selon la configuration du système) ;
Fprec : la force générée par le système de précontrainte ;
K : la raideur lié au réservoir à volume variable (cas d'un soufflet par exemple), celle-ci pouvant être négligée dans certaines configurations du réservoir à volume variable (cas d'un système à membrane roulante ou d'un piston) ;
i : la position relative de la partie mobile du réservoir à volume variable par rapport à une position de référence,
Fpertes : les pertes mécaniques liées aux frottements des différentes pièces mécaniques lors de la transmission des forces sur le capteur de force
Fcapteur : la force mesurée par le ou les capteurs de force
Pboîtier : la pression du gaz contenu dans le boîtier hermétique implantable, ladite pression pouvant soit être déduite de la position de la partie mobile par rapport à sa position de référence et de la surface effective de pression de la partie mobile, soit être mesurée par un capteur de pression de gaz placé dans le boîtier (optionnel).

A partir de ces éléments on peut en déduire plusieurs forces appliquées sur la paroi mobile du réservoir à volume variable :

Foccl : la force (positive ou négative) ramenée sur la partie mobile du réservoir à volume variable liée à la pression dans le circuit fluidique du système occlusif ;
Fboitier : la force (positive ou négative) ramenée sur la partie mobile du réservoir à volume variable liée à la pression dans le boitier hermétique implantable ;
Fparoi : la force liée à la position et à la raideur de la partie mobile du réservoir à volume variable.

On considère par la suite que la pression de gaz dans le boîtier est déduite de la position de la partie mobile par rapport à sa position de référence et de la surface effective de pression de la partie mobile.

Le bilan des forces sur le capteur de force se traduit de la manière suivante :

$$Fcapteur = Foccl + Fprec - Fparoi - Fboitier - Fpertes$$

avec :

$$Foccl = P \cdot Seff$$

$$Fparoi = K \cdot i$$

$$Fboitier = Pinit \cdot \left(\frac{Vinit}{Vinit - Seff \cdot i} - 1\right) \cdot Seff$$

avec Pinit et Vinit des constantes correspondant respectivement à la pression initiale et au volume initial de gaz dans le boîtier lorsque celui-ci a été hermétiquement scellé.

Pinit peut être par exemple égale à la pression atmosphérique lorsque le boîtier est hermétiquement scellé.

La force générée par le dispositif de précontrainte permet de créer un « offset » sur le capteur de force ce qui permet de mesurer des forces positives et négatives.

La force Fboitier est déduite à partir de la position de la paroi mobile par rapport à son origine et de la surface effective de pression de la paroi mobile. On considère dans ce cas que le boîtier est parfaitement hermétique. On peut également supposer une perte négligeable de gaz du boîtier vers l'extérieur qui peut être soit négligée soit prise en compte dans le calcul de la force Fboîtier. Enfin, la force Fboîtier peut être également directement mesurée par un capteur de pression et déduite en multipliant par la surface Seff.

On peut donc en déduire la pression P1 du circuit fluidique du système occlusif :

$$P1 = \frac{Fcapteur - Fprec + Fparoi + Fboitier + Fpertes}{Seff}$$

$$P1 = \frac{Fcapteur - Fprec + K \cdot i + Fpertes}{Seff} + Pinit \cdot \left(\frac{Vinit}{Vinit - Seff \cdot i} - 1\right)$$

avec Fprec et Fpertes des constantes connues.

La force Fprec peut être générée par des ressorts précontraints. Le déplacement du bloc 15 étant très faible, on peut considérer que la course des ressorts est négligeable par rapport à leur raideur et que la force de précontrainte est constante.

Fprec est choisi de sorte à pouvoir mesurer des pressions négatives et positives dans toute la gamme de mesure désirée.

Dans le cas où le capteur de force est capable de mesurer des forces de compression et de traction, il est possible de retirer le système de précontrainte (cas des modes de réalisation décrits dans la figure 3 et la figure 5). Dans ce cas, la constante Fprec n'est pas considérée dans les calculs de P1.

Afin de déduire la pression relative P2 dans le circuit fluidique, la pression atmosphérique courante exercée sur le corps du patient doit être prise en compte. La mesure de la pression Patm peut être réalisée par exemple comme il a été décrit dans les paragraphes précédents.

Dans ce cas, P2 est calculée de la manière suivante :

$$P2 = P1 + (Pinit - Patm)$$

Dans un mode de réalisation préféré, le boîtier est scellé de manière à maximiser le volume d'air dans le boitier (paroi mobile du réservoir en position initiale de sorte à avoir un volume minimal dans le réservoir à volume variable). Cela permet d'avoir une pression du gaz dans le boitier toujours positive quelle que soit la position de la paroi mobile.

Les paragraphes ci-dessous présentent deux exemples de mesure de la pression à partir de la méthode décrite dans la présente invention.

Le dispositif décrit dans les deux exemples ci-dessous a les caractéristiques suivantes :

- Seff = $1 \times 10^{-3}$ m$^2$
- K = 5N/mm

- Fprec = 20 N
- Vinit = 10x10$^{-5}$ m$^3$
- Pinit = 1000 hPa
- Patm = 900 hPa
- Fpertes = 0 N

Nous avons considéré dans ces exemples que la pression atmosphérique lors du scellage du boîtier (Pinit) est différente de la pression atmosphérique (Patm) exercée sur le corps du patient lors de la mesure de la pression dans le circuit fluidique.

Exemple 1 : mesure de la pression dans le circuit fluidique à partir d'un dispositif selon le mode de réalisation décrit dans la figure 2, comprenant un capteur de force mesurant uniquement des forces de compression.

Pour i = 0 (position haute du soufflet, correspondant à un volume du réservoir minimum), l'unité de traitement réalise le calcul suivant pour mesurer la pression dans le circuit fluidique :

$$P1 = \frac{Fcapteur - 20}{1x10^{-3}}$$

Pour une pression relative nulle dans le circuit fluidique par exemple, le capteur de force mesure la force de compression de la précontrainte (20 N) ainsi que la force liée à la différence de pression atmosphérique (900 hPa) exercée sur le corps du patient, et donc sur la manchette occlusive souple, et la pression de gaz dans le boîtier (1000 hPa), soit une valeur P1 calculée par l'unité de traitement :

$$Fcapteur = 20\,N + Seff \times (Patm - Pinit)$$

$$Fcapteur = 20\,N + 1.10^{-3} \times (90000 - 100000) = 10\,N$$

$$P1 = \frac{10 - 20}{1 \times 10^{-3}} = -10\ kPa$$

On peut en déduire la pression relative P2 dans le circuit fluidique :

$$P2 = P1 + (Pinit - Patm)$$

$$P2 = -10000 + (100000 - 90000) = 0\ Pa$$

Pour une pression relative dans le circuit fluidique de 5 kPa par exemple, la force sur le capteur de force est celle générée par la pression dans le circuit fluidique Foccl (soit 5 N), celle de la précontrainte (soit 20 N) et celle liée à la pression atmosphérique (-10 N), soit une force résultante de 15 N, soit une valeur calculée par l'unité de traitement :

$$P1 = \frac{15 - 20}{1 \times 10^{-3}} = -5\ kPa$$

On peut en déduire la pression relative P2 dans le circuit fluidique :

$$P2 = P1 + (Pinit - Patm)$$

*P2* = -5000 + (100000 - 90000) = 5 *kPa*Pour une pression relative dans le circuit fluidique de -5 kPa par exemple, la force sur le capteur de force est celle générée par la pression dans le circuit fluidique Foccl (soit -5 N), celle de la précontrainte (soit 20 N) et celle liée à la pression atmosphérique (soit -10 N), soit une force résultante de 5 N, soit une valeur calculée par l'unité de traitement :

$$P1 = \frac{5 - 20}{1 \times 10^{-3}} = -15\, kPa$$

On peut en déduire la pression relative P2 dans le circuit fluidique :

$$P2 = P1 + (Pinit - Patm)$$

$$P2 = -15000 + (100000 - 90000) = -5\, kPa$$

Pour i = 4 mm par exemple (position basse du soufflet, générant une compression de gaz dans le boîtier et une force de rappel liée à la raideur du soufflet), l'unité de traitement réalise le calcul suivant pour mesurer la pression dans le circuit fluidique :

$$P1 = \frac{Fcapteur - 5 \times 4 + 20}{1 \times 10^{-3}} + 100000 \times \left(\frac{10 \cdot 10^{-5}}{10 \cdot 10^{-5} - 1 \cdot 10^{-3} \times 4 \cdot 10^{-3}} - 1\right)$$

$$P1 = \frac{Fcapteur}{1 \times 10^{-3}} + 100000 \times \left(\frac{10 \cdot 10^{-5}}{10 \cdot 10^{-5} - 1 \cdot 10^{-3} \times 4 \cdot 10^{-3}} - 1\right)$$

$$P1 = \frac{Fcapteur}{1 \times 10^{-3}} + 4166,67$$

Pour une pression relative nulle dans le circuit fluidique par exemple, les forces sur le capteur de force sont Fprec (20 N), Fparoi (-20 N), Fboîtier (-4,167 N) et la force liée à la pression atmosphérique (-10 N), soit une force résultante de -14,167 N, d'où une valeur calculée par l'unité de traitement :

$$P1 = \frac{-14,167}{1 \times 10^{-3}} + 4166,67 = -10\, kPa$$

On peut en déduire la pression relative P2 dans le circuit fluidique :

$$P2 = P1 + (Pinit - Patm)$$

$$P2 = -10000 + (100000 - 90000) = 0\, Pa$$

Pour une pression relative dans le circuit fluidique de 5 kPa par exemple, les forces sur le capteur de force sont Foccl (5 N), Fprec (20 N), Fparoi (-20 N), Fboîtier (-4,167 N) et la force liée à la pression atmosphérique (-10 N), soit une force résultante de -9,167 N, d'où une valeur calculée par l'unité de traitement :

$$P1 = \frac{-9,167}{1 \times 10^{-3}} + 4166,67 = -5\, kPa$$

On peut en déduire la pression relative P2 dans le circuit fluidique :

$$P2 = P1 + (Pinit - Patm)$$

$$P2 = -5000 + (100000 - 90000) = 5\, kPa$$

Pour une pression relative dans le circuit fluidique de -5 kPa par exemple, les forces sur le capteur de force sont Foccl (-5 N), Fprec (20 N), Fparoi (-20 N), Fboîtier (-4,167 N) et la force liée à la pression atmosphérique (-10 N), soit une force résultante de -19,167N, d'où une valeur calculée par l'unité de traitement :

$$P1 = \frac{-19,167}{1 \times 10^{-3}} + 4166,67 = -15\, kPa$$

On peut en déduire la pression relative P2 dans le circuit fluidique :

$$P2 = P1 + (Pinit - Patm)$$

$$P2 = -15000 + (100000 - 90000) = -5\, kPa$$

Exemple 2 : mesure de la pression dans le circuit fluidique à partir d'un dispositif basé sur le mode de réalisation décrit dans la figure 5, comprenant un capteur de force capable de mesurer des forces de compression et des forces de tractions.
Pour i = 0, l'unité de traitement réalise le calcul suivant pour mesurer la pression dans le circuit fluidique :

$$P1 = \frac{Fcapteur}{1 \times 10^{-3}}$$

Pour une pression relative nulle dans le circuit fluidique par exemple, le capteur de force mesure uniquement une force liée à la différence de pression atmosphérique (900 hPa) exercée sur le corps du patient, et donc sur la manchette occlusive souple, et la pression de gaz dans le boîtier (1000 hPa), correspondant à une force de -10 N, soit une valeur calculée par l'unité de traitement :

$$P1 = \frac{-10}{1 \times 10^{-3}} = -10\, kPa$$

On peut en déduire la pression relative P2 dans le circuit fluidique :

$$P2 = P1 + (Pinit - Patm)$$

$$P2 = -10000 + (100000 - 90000) = 0\, Pa$$

Pour une pression relative dans le circuit fluidique de 5 kPa par exemple, la seule force sur le capteur de force est celle générée par la pression dans le circuit fluidique Foccl (soit 5 N) et la force liée à la pression atmosphérique (soit -10 N), soit une valeur calculée par l'unité de traitement :

$$P1 = \frac{-5}{1 \times 10^{-3}} = -5\, kPa$$

On peut en déduire la pression relative P2 dans le circuit fluidique :

$$P2 = P1 + (Pinit - Patm)$$

$$P2 = -5000 + (100000 - 90000) = 5\ kPa$$

Pour une pression relative dans le circuit fluidique de -5 kPa par exemple, la seule force sur le capteur de force est celle générée par la pression dans le circuit fluidique Foccl (soit -5 N) et la force liée à la pression atmosphérique (soit -10 N), soit une valeur calculée par l'unité de traitement :

$$P1 = \frac{-15}{1 \times 10^{-3}} = -15\ kPa$$

On peut en déduire la pression relative P2 dans le circuit fluidique :

$$P2 = P1 + (Pinit - Patm)$$

$$P2 = -15000 + (100000 - 90000) = -5\ kPa$$

Pour i = 4 mm, l'unité de traitement réalise le calcul suivant pour mesurer la pression dans le circuit fluidique :

$$P1 = \frac{Fcapteur + 5 \times 4}{1 \times 10^{-3}} + 100000 \times \left( \frac{10 \cdot 10^{-5}}{10 \cdot 10^{-5} - 1 \cdot 10^{-3} \times 4 \cdot 10^{-3}} - 1 \right)$$

$$P1 = \frac{Fcapteur + 20}{1 \times 10^{-3}} + 4166,67$$

Pour une pression relative nulle dans le circuit fluidique par exemple, les forces sur le capteur de force sont Fparoi (-20 N) et Fboîtier (-4,167 N), et la force liée à la pression atmosphérique (-10 N), soit une force résultante de -34,167 N, soit une valeur calculée par l'unité de traitement :

$$P1 = \frac{-34,167 + 20}{1 \times 10^{-3}} + 4166,67 = -10\ kPa$$

On peut en déduire la pression relative P2 dans le circuit fluidique :

$$P2 = P1 + (Pinit - Patm)$$

$$P2 = -10000 + (100000 - 90000) = 0\ Pa$$

Pour une pression relative dans le circuit fluidique de 5 kPa par exemple, les forces sur le capteur de force sont Foccl (5 N), Fparoi (-20 N) et Fboîtier (-4,167 N), et la force liée à la pression atmosphérique (-10 N), soit une force résultante de -29,167 N, soit une valeur calculée par l'unité de traitement :

$$P1 = \frac{-29,167 + 20}{1 \times 10^{-3}} + 4166,67 = -5\ kPa$$

On peut en déduire la pression relative P2 dans le circuit fluidique :

$$P2 = P1 + (Pinit - Patm)$$

$$P2 = -5000 + (100000 - 90000) = 5\ kPa$$

Pour une pression relative dans le circuit fluidique de -5 kPa par exemple, les forces sur le capteur de force sont Foccl (-5 N), Fparoi (-20 N) et Fboîtier (-4,167 N) et la force liée à la pression atmosphérique (-10 N), soit une force résultante de -39,167 N, soit une valeur calculée par l'unité de traitement :

$$P1 = \frac{-39{,}167 + 20}{1 \times 10^{-3}} + 4166{,}67 = -15\ kPa$$

On peut en déduire la pression relative P2 dans le circuit fluidique :

$$P2 = P1 + (Pinit - Patm)$$

$$P2 = -15000 + (100000 - 90000) = -5\ kPa$$

Dans les deux exemples ci-dessus, il est supposé que le dispositif ne comprend pas de capteur de pression mesurant la pression de gaz dans le boîtier.

Dans une configuration où le dispositif comprend un capteur de pression mesurant la pression de gaz dans le boitier, il suffit de remplacer le terme

$$Fboitier = Pinit \cdot \left( \frac{Vinit}{Vinit - Seff \cdot i} - 1 \right) \cdot Seff \ \text{par} \ Pcapteur$$

avec Pcapteur, la valeur de mesure du capteur de pression de gaz.

Il est à noter que la présente invention peut être également appliquée à des dispositifs comportant un réservoir à volume variable sans raideur mécanique de type piston ou membrane roulante. Dans ce cas, la raideur K dans le calcul de la pression P1 est considérée nulle ou négligeable.

Contrôle en pression du système occlusif

Un objectif préféré du système étant de contrôler la pression, une régulation en boucle fermée de la pression paraît à première vue être le choix le plus judicieux pour la pressurisation du système occlusif. Cependant, la pression dans le système occlusif n'est pas uniquement dépendante du système de pressurisation. Des effets extérieurs liés aux mouvements des organes du patient ou à sa respiration par exemple peuvent générer des pressions qui seront mesurées par le système de mesure de pression décrit précédemment.

Cela peut avoir pour effet, lors de l'asservissement en pression, d'induire une sollicitation excessive de l'actionneur et donc une consommation électrique trop importante. En effet, les pressions extérieures pouvant varier lors d'un changement de pression du système occlusif, le système d'asservissement aura tendance à chercher à compenser ses variations, ce qui engendrera une sollicitation excessive de l'actionneur.

Pour pallier ce problème, l'un des objets de cette invention est de proposer une méthode simple de contrôle en pression du système de pressurisation. Plutôt que d'asservir le système en pression, l'actionneur est contrôlé par un asservissement en position de la partie mobile du réservoir à volume variable. Connaissant la surface de pression effective de la partie mobile à chaque position, cela correspond donc à un asservissement en volume du système d'occlusion.

Des tests réalisés in vitro et in vivo ([1]) ont montré que la relation entre la pression dans le circuit fluidique et le volume injecté dans le système occlusif ont une relation définie et répétable dans le temps. Cela est vrai dans des conditions particulières du patient, c'est-à-dire quand celui-ci est immobile et dans une position déterminée (allongé ou debout par exemple).

Le dispositif de contrôle de la pression de fluide dans le circuit fluidique comprend notamment une mémoire dans laquelle est enregistrée une relation entre la pression dans le circuit fluidique et le volume injecté à partir du ou vers le réservoir, une unité de traitement (éventuellement identique à l'unité de traitement du dispositif de mesure de la pression dans le circuit fluidique) et une unité de calibration, dont le fonctionnement est décrit ci-après.

La figure 8 est un graphe illustrant la variation de la pression dans le circuit fluidique en fonction du volume injecté dans le circuit fluidique à partir du réservoir. Il est à noter que la relation de pression en fonction du volume injecté ou retiré peut présenter une hystérésis, c'est-à-dire que la courbe de pression en montée en pression peut être différente de la courbe en descente de pression. Pour pouvoir contrôler le système en pression, le système de pressurisation effectue régulièrement des mesures de pression pour des volumes d'injection donnés. Cette procédure de calibration est réalisée dans des conditions prédéterminées. Par exemple, dans le cas d'un sphincter urinaire artificiel, la calibration peut être réalisée quelques minutes après la miction et lorsque le patient est debout et sensiblement immobile. A cet effet, le système de pressurisation augmente graduellement la pression pour des volumes d'injections prédéterminés et enregistre les valeurs mesurées dans une table localisée dans la mémoire de l'implant. Cette procédure de calibration peut être réalisée à une période définie, par exemple hebdomadairement, ou bien lors de consultation chez le médecin, à l'aide d'un programmateur communiquant sans fil avec l'implant. Dans tous les cas, la pression atmosphérique courante doit pouvoir être récupérée pour effectuer une mesure de pression précise dans le circuit fluidique, quel que soit l'altitude ou les conditions météorologiques du lieu où se trouve le patient lors de la calibration.

La figure 9 est un graphe illustrant la variation 28 de la pression P2 dans le circuit fluidique en fonction du temps t pour différents volumes de fluide injectés dans la manchette occlusive lors de la procédure de calibration.

La calibration peut être réalisée en plus lorsque le patient est en position allongée et sensiblement immobile. Cela permet d'enregistrer des valeurs de pression qui seront différentes de celles enregistrées lorsque le patient est debout, du fait de la colonne d'eau entre le boîtier implantable et la manchette occlusive implantés à des hauteurs différentes dans le patient.

La calibration peut prendre en compte l'hystérésis qui pourrait exister dans le circuit fluidique. Dans ce cas, la mémoire de l'implant comporte une table contenant les valeurs de volume à injecter pour atteindre une pression donnée et ainsi que les valeurs de volume à retirer pour atteindre une pression donnée.

En fonctionnement normal, lorsqu'une commande de pressurisation à une pression donnée est envoyée au système de pressurisation, le volume correspondant à la pression de consigne est recherché dans la table en mémoire et il est utilisé pour pressuriser le système d'occlusion à la pression désirée.

Par mesure de sécurité, la pression peut être mesurée lors de la phase de pressurisation afin de s'assurer du bon fonctionnement du dispositif et d'une correspondance avec les valeurs de pression attendues.

Pour mesurer les mouvements du patient et déterminer s'il est immobile, et pour mesurer sa posture et déterminer s'il est debout ou allongé, un accéléromètre peut être utilisé.

Pour réaliser la procédure de calibration du volume en fonction de la pression dans le système d'occlusion, une horloge est utilisée. Elle pourra être à titre d'exemple de type RTC.

Système de sécurité

Dans le cas où la pression dans le circuit fluidique devient très élevée et proche des limites définies dans les préconisations techniques concernant la tenue en pression des différents éléments du circuit fluidique, l'unité de traitement peut envoyer de manière automatique un ordre de décompression de la manchette occlusive.

Selon un mode de réalisation particulièrement avantageux de l'invention, le dispositif d'actionnement de la manchette comprend un organe de réduction des contraintes mécaniques causées par une pression trop forte dans le circuit fluidique, permettant de protéger le dispositif d'actionnement des risques de détérioration.

Les contraintes mécaniques subies par le dispositif d'actionnement résultant d'une pression trop forte dans le circuit fluidique peuvent devenir très importantes, ce qui pourrait causer la détérioration de l'une ou plusieurs des parties du dispositif d'actionnement. Le système de pressurisation, la tubulure, la manchette, le capteur de pression et/ou les connecteurs peuvent être concernés par cette dégradation.

Afin d'éviter de détériorer le dispositif d'actionnement, l'organe de réduction des contraintes est conçu pour, lorsque les contraintes mécaniques (pression dans le circuit fluidique) dépassent un seuil de contraintes déterminé, absorber une partie desdites contraintes de sorte à réduire les contraintes subies par le dispositif d'actionnement.

L'organe de réduction des contraintes est dimensionné pour diminuer les contraintes jusqu'à un niveau dans lequel elles sont trop faibles pour risquer d'endommager le dispositif d'actionnement tout en étant suffisamment élevées pour ne pas relâcher totalement la compression exercée par la manchette.

En fonction du mode de réduction des contraintes envisagé et de la structure du dispositif d'actionnement, l'homme du métier est en mesure de concevoir un organe remplissant ces conditions.

Selon un mode de réalisation, l'organe de réduction des contraintes comprend une chambre d'expansion agencée dans le circuit hydraulique et se déclenchant mécaniquement lorsque la pression dans le circuit fluidique dépasse un seuil défini. Cela a pour effet de transférer une partie du fluide du circuit hydraulique vers la chambre d'expansion afin de réduire la pression dans ce dernier.

De manière alternative, l'organe de réduction des contraintes peut comprendre un piston couplé à un système de ressort ayant une raideur suffisamment élevée pour rester sensiblement fixe lorsque la pression dans le circuit hydraulique correspond à la pression de fonctionnement normal du dispositif et mobile sous l'effet d'une pression plus élevée.

L'organe de réduction des contraintes peut présenter différentes formes d'exécution ; par exemple et de manière non limitative :

- un clapet couplé avec un ressort ou un matériau spécifique dans une chambre d'expansion ;
- un composant du circuit hydraulique réalisé en un matériau souple ayant la propriété de se déformer à partir d'un certain seuil de pression ;
- une membrane déformable au-delà d'un certain seuil de pression ou en fonction de la pression appliquée ;
- le réservoir à volume variable conçu de manière à être déformable au-delà d'un certain seuil de pression ou en fonction de la pression appliquée ;
- le mécanisme d'actionnement conçu de manière à être déformable au-delà d'un certain seuil de pression ou en fonction de la pression appliquée.

## REFERENCES

EP 1 584 303
US 8,585,580
US 4,581,018
US 4,222,377
CA 1 248 303
US 4,408,597

[1] Lamraoui, H ; Bonvilain, A ; Robain, G ; Combrisson, H ; Basrour, S ; Moreau-Gaudry, A ; Cinquin, P ; Mozer, P "Development of a Novel Artificial Urinary Sphincter: A Versatile Automated Device", IEEE - ASME Transactions on Mechatronics, 15, 916-924, 2010.

## Revendications

1. Système occlusif implantable dans un corps humain ou animal, comprenant :

    - un circuit fluidique comprenant :

        • une manchette occlusive (3) gonflable contenant un volume variable d'un fluide, destinée à entourer au moins une partie d'un conduit naturel à occlure,
        • un réservoir (5) à volume variable rempli d'un fluide, ledit réservoir comprenant une partie fixe et une partie mobile,
        • une liaison fluidique (2) entre le réservoir (5) et la manchette occlusive (3),

    - un actionneur couplé mécaniquement à la partie mobile du réservoir de sorte à déplacer linéairement ladite partie mobile par rapport à la partie fixe pour ajuster le volume du réservoir,

    l'actionneur et le réservoir à volume variable étant agencés dans un boîtier (1) étanche contenant un gaz,
    ledit système étant **caractérisé en ce qu'**il comprend en outre :

        - un capteur (21, 22) agencé dans le boîtier (1), lié mécaniquement à l'actionneur et/ou à la partie mobile du réservoir, agencé de sorte à mesurer une force de traction et/ou de compression dans la direction de déplacement de la partie mobile du réservoir, ladite force mesurée résultant au moins de :

        • la force ($F_{occl}$) exercée sur la partie mobile du réservoir à volume variable liée à la pression dans le circuit fluidique, et
        • la force ($F_{boîtier}$) exercée sur la partie mobile du réservoir à volume variable liée à la pression de gaz dans le boîtier,

        - un dispositif de mesure de la pression (P1) de fluide dans le circuit fluidique comprenant une unité de traitement configurée pour déterminer ladite pression (P1) de fluide à partir d'un calcul prenant en compte au moins la mesure de force dudit capteur (21, 22), la surface de pression effective (Seff) de la partie mobile du réservoir, et la force (Fboîtier) exercée sur la partie mobile du réservoir à volume variable liée à la pression de gaz dans le boîtier.

2. Système selon la revendication 1, comprenant en outre un dispositif de contrôle de la pression de fluide dans le circuit fluidique par le volume du réservoir, comprenant :

• une mémoire dans laquelle est enregistrée une relation entre la pression dans le circuit fluidique et le volume dudit réservoir,
• une unité de traitement configurée pour :

- recevoir une consigne de pression de fluide dans le circuit fluidique,
- à partir de la relation enregistrée dans la mémoire entre la pression dans le réservoir et le volume du réservoir, déterminer le volume du réservoir permettant d'atteindre la pression de consigne,
- le cas échéant, commander l'actionneur pour déplacer la partie mobile du réservoir dans la position définissant ledit volume déterminé,

• une unité de calibration configurée pour :

(a) lorsque le patient est dans une situation déterminée, commander l'actionneur pour déplacer la partie mobile du réservoir dans une pluralité de positions déterminées, chaque position définissant un volume déterminé du réservoir,
(b) pour chacune desdites positions :

- mesurer la pression (P1) de fluide dans le circuit fluidique par ledit dispositif de mesure de la pression de fluide dans le circuit fluidique,
- mettre à jour la mémoire en enregistrant ladite pression de fluide mesurée dans le circuit fluidique pour le volume respectif du réservoir.

3. Système selon l'une des revendications 1 ou 2, dans lequel ledit capteur est apte à mesurer des forces de traction et des forces de compression dans la direction de déplacement de la partie mobile du réservoir.

4. Système selon l'une des revendications 1 ou 2, dans lequel ledit capteur est apte à mesurer uniquement des forces de compression dans la direction de déplacement de la partie mobile du réservoir, ledit système comprenant en outre un dispositif de précontrainte agencé de sorte à exercer une précontrainte de compression déterminée sur ledit capteur.

5. Système selon la revendication 4, dans lequel l'unité de traitement est configurée pour prendre en compte ladite précontrainte pour la détermination de la pression (P1) de fluide dans le circuit fluidique.

6. Système selon l'une des revendications 4 ou 5, dans lequel ledit dispositif de précontrainte comprend au moins un ressort de compression, un ressort de traction et/ou un plot en élastomère.

7. Système selon l'une des revendications 1 à 6, dans lequel la partie mobile du réservoir à volume variable comprend un système d'entraînement (10, 17) couplé à une paroi mobile (6) et un soufflet déformable s'étendant et se comprimant en fonction de la position de ladite paroi mobile.

8. Système selon la revendication 7, dans lequel l'unité de traitement est configurée pour prendre en compte la raideur dudit soufflet pour la détermination de la pression (P1) de fluide dans le circuit fluidique.

9. Système selon l'une des revendications 7 ou 8, dans lequel :

- le système d'entraînement comprend une vis (17) solidaire de la paroi mobile (6) et une noix (10) couplée à la vis (17) et mobile en rotation sur une butée à billes (16) à double effet autour de l'axe de la vis sous l'effet d'une action d'entraînement par l'actionneur, la noix (10) étant couplée à la vis (17) de sorte qu'une rotation de la noix (10) entraîne la vis (17) uniquement en translation dans la direction de déplacement de la partie mobile (6),
- le capteur (22) est agencé dans ladite butée à billes (16) de sorte à mesurer au moins une force de traction et une force de compression dans la direction de déplacement de la partie mobile du réservoir.

10. Système selon l'une des revendications 1 à 6, dans lequel la partie mobile du réservoir à volume variable comprend un système d'entraînement (17) couplé à une membrane roulante.

11. Système selon l'une des revendications 1 à 6, dans lequel le réservoir à volume variable comprend un cylindre formant la partie fixe du réservoir et un piston coulissant dans ledit cylindre, formant la partie mobile du réservoir.

12. Système selon l'une des revendications 1 à 11, dans lequel l'actionneur est choisi parmi les actionneurs piézoélectriques, les actionneurs électromagnétiques, les polymères électro-actifs et les alliages à mémoire de forme.

13. Système selon l'une des revendications 1 à 12, comprenant en outre un capteur de pression de gaz agencé dans le boîtier (1) pour la mesure de la pression de gaz dans le boîtier, l'unité de traitement étant configurée pour prendre en compte ladite pression de gaz mesurée dans la détermination de la force ($F_{boîtier}$).

14. Système selon l'une des revendications 1 à 13, dans lequel une paroi du réservoir à volume variable est constituée par une paroi du boîtier, ladite paroi comprenant un port (4) de ponction perforable.

15. Système selon l'une des revendications 1 à 14, comprenant en outre un dispositif de réduction des contraintes dans le circuit fluidique lorsque lesdites contraintes dépassent un seuil déterminé.

16. Système selon l'une des revendications 1 à 15, comprenant en outre un accéléromètre, l'unité de traitement étant configurée pour, à partir des données de mesure de l'accéléromètre, déterminer si le patient est dans une situation déterminée.

17. Système selon l'une des revendications 1 à 16, dans lequel le dispositif de mesure de la pression est configuré pour mesurer la pression de fluide lors de l'ajustement du volume du réservoir et pour vérifier la correspondance entre ladite valeur mesurée et une valeur attendue.

18. Système selon l'une des revendications 1 à 17, consistant en un sphincter urinaire artificiel.

19. Système selon l'une des revendications 1 à 18, dans lequel l'unité de traitement est configurée pour calculer une pression relative (P2) de fluide dans le circuit fluidique, ladite pression relative étant égale à la différence entre la pression de fluide (P1) déterminée dans le circuit fluidique et la pression atmosphérique (Patm) s'exerçant sur le patient.

20. Système selon la revendication 19, comprenant en outre un dispositif destiné à être placé à l'extérieur du corps du patient et comprenant un capteur barométrique adapté pour mesurer la pression atmosphérique s'exerçant sur le patient, ledit dispositif étant capable de communiquer ladite mesure de pression à l'unité de traitement pour le calcul de ladite pression relative du fluide dans le circuit fluidique.

21. Système selon la revendication 19, dans lequel l'unité de traitement est configurée pour déterminer la pression atmosphérique s'exerçant sur le patient à partir de la pression dans le circuit de fluide et d'une pression relative connue dans une configuration d'actionnement donnée de la manchette.

**Patentansprüche**

1. Okklusives System, das in einen menschlichen oder tierischen Körper implantierbar ist und Folgendes umfasst:

   - einen Fluidkreislauf umfassend:

     • eine aufblasbare Verschlussmanschette (3), die ein variables Volumen eines Fluids enthält und dazu bestimmt ist, zumindest einen Teil eines zu verschließenden natürlichen Gangs zu umgeben,
     • einen mit einem Fluid gefüllten Behälter (5) mit variablem Volumen, wobei der Behälter einen fixen Teil und einen mobilen Teil umfasst,
     • eine Fluidverbindung (2) zwischen dem Behälter (5) und der Verschlussmanschette (3),

   - ein Betätigungselement, das mechanisch mit dem mobilen Teil des Behälters gekoppelt ist, um den mobilen Teil geradlinig relativ zum fixen Teil zu bewegen und das Volumen des Behälters einzustellen,

wobei das Betätigungselement und der volumenvariable Behälter in einem abgedichteten Gehäuse (1) angeordnet sind, das ein Gas enthält,
wobei das System **dadurch gekennzeichnet ist, dass** es ferner umfasst:

- einen im Gehäuse (1) angeordneten Sensor (21, 22), der mechanisch mit dem Betätigungselement und/oder dem mobilen Teil des Behälters verbunden und so gestaltet ist, dass er eine Zug- und/oder Druckkraft in Bewegungsrichtung des mobilen Teils des Behälters misst, wobei die gemessene Kraft zumindest aus Folgendem besteht:

- die Kraft ($F_{occl}$), die auf den mobilen Teil des Behälters mit variablem Volumen, bezogen auf den Druck im Fluidkreislauf, ausgeübt wird, und
- die Kraft ($F_{Gehäuse}$), die auf den mobilen Teil des Behälters mit variablem Volumen, bezogen auf den Gasdruck im Gehäuse, ausgeübt wird,

- eine Fluiddruckmessvorrichtung (P1) im Fluidkreislauf, die eine Verarbeitungseinheit umfasst, die konfiguriert ist, um den Fluiddruck (P1) ausgehend von einer Berechnung zu bestimmen, bei der zumindest die Kraftmessung des Sensors (21, 22), die effektive Druckfläche (Seff) des mobilen Teils des Behälters und die Kraft ($F_{Gehäuse}$) berücksichtigt werden, die auf den mobilen Teil des Behälters mit variablem Volumen, bezogen auf den Gasdruck im Gehäuse, ausgeübt wird.

2. System nach Anspruch 1, das ferner eine Vorrichtung zur Steuerung des Fluiddrucks im Fluidkreislauf durch das Volumen des Behälters aufweist, umfassend:

- einen Speicher, in dem eine Beziehung zwischen dem Druck im Fluidkreislauf und dem Volumen des Behälters aufgezeichnet ist,
- eine Verarbeitungseinheit, die für Folgendes konfiguriert ist:

- einen Fluiddruck-Sollwert im Fluidkreislauf erhalten,
- ausgehend vom im Speicher aufgezeichneten Verhältnis zwischen dem Druck im Behälter und dem Volumen des Behälters das Volumen des Behälters bestimmen, mit dem der Drucksollwert erreicht werden kann,
- gegebenenfalls das Betätigungselement steuern, um den mobilen Teil des Behälters in die Position zu bringen, die das bestimmte Volumen definiert,

- eine Kalibriereinheit, die wie folgt konfiguriert ist:

(a) wenn sich der Patient in einer bestimmten Lage befindet, Steuern des Betätigungselements, um den mobilen Teil des Behälters in eine Vielzahl von bestimmten Positionen zu bewegen, wobei jede Position ein bestimmtes Volumen des Behälters definiert,
(b) bei jeder dieser Positionen:

- Messen des Fluiddrucks (P1) im Fluidkreislauf durch die Fluiddruckmessvorrichtung im Fluidkreislauf,
- Aktualisieren des Speichers durch Aufzeichnen des im Fluidkreislauf für das jeweilige Behältervolumen gemessenen Fluiddrucks.

3. System nach einem beliebigen der vorstehenden Ansprüche 1 oder 2, wobei der Sensor in der Lage ist, Zug- und Druckkräfte in Verschiebungsrichtung des mobilen Teils des Behälters zu messen.

4. System nach einem beliebigen der vorstehenden Ansprüche 1 oder 2, wobei der Sensor in der Lage ist, nur Druckkräfte in Verschiebungsrichtung des mobilen Teils des Behälters zu messen, wobei das System ferner eine Vorspannvorrichtung umfasst, die so gestaltet ist, dass sie eine vorbestimmte Druckvorspannung auf den Sensor ausübt.

5. System nach Anspruch 4, wobei die Verarbeitungseinheit so konfiguriert ist, dass sie bei der Bestimmung des Fluiddrucks (P1) im Fluidkreislauf die Vorspannung berücksichtigt.

6. System nach einem beliebigen der vorstehenden Ansprüche 4 oder 5, wobei die Vorspannvorrichtung zumindest eine Druckfeder, eine Zugfeder und/oder einen Elastomerplot aufweist.

7. System nach einem beliebigen der vorstehenden Ansprüche 1 bis 6, wobei der mobile Teil des Behälters mit variablem Volumen ein Antriebssystem (10, 17) umfasst, das mit einer mobilen Wand (6) gekoppelt ist, und einen verformbaren Balg, der sich je nach der Position der mobilen Wand ausweitet und zusammenzieht.

8. System nach Anspruch 7, wobei die Verarbeitungseinheit so konfiguriert ist, dass sie bei der Bestimmung des Fluiddrucks (P1) im Fluidkreislauf die Steifigkeit des Balgs berücksichtigt.

9. System nach einem beliebigen der vorstehenden Ansprüche 7 oder 8, wobei:

- das Antriebssystem eine mit der mobilen Wand (6) fest verbundene Schraube (17) und eine mit der Schraube (17) gekoppelte Mutter (10) aufweist, die auf einem zweiseitig wirkenden Kugellager (16) unter der Wirkung einer Antriebsaktion durch das Betätigungselement um die Achse der Schraube in Drehung mobil ist, wobei die Mutter (10) mit der Schraube (17) gekoppelt ist, so dass eine Drehung der Mutter (10) die Schraube (17) nur in Verschiebungsrichtung des mobilen Teils (6) antreibt,
- der Sensor (22) so im Kugellager (16) angeordnet ist, dass er zumindest eine Zugkraft und eine Druckkraft in Verschiebungsrichtung des mobilen Teils des Behälters misst.

10. System nach einem beliebigen der vorstehenden Ansprüche 1 bis 6, wobei der mobile Teil des Behälters mit variablem Volumen ein Antriebssystem (17) umfasst, das mit einer Rollmembran gekoppelt ist.

11. System nach einem beliebigen der vorstehenden Ansprüche 1 bis 6, wobei der Behälter mit variablem Volumen einen Zylinder umfasst, der den fixen Teil des Behälters bildet, und einen im Zylinder gleitenden Kolben, der den mobilen Teil des Behälters bildet.

12. System nach einem beliebigen der vorstehenden Ansprüche 1 bis 11, wobei das Betätigungselement unter piezoelektrischen Betätigungselementen, elektromagnetischen Betätigungselementen, elektroaktiven Polymeren und Formgedächtnislegierungen ausgewählt ist.

13. System nach einem beliebigen der vorstehenden Ansprüche 1 bis 12, das ferner einen im Gehäuse (1) gestalteten Gasdrucksensor zur Messung des Gasdrucks im Gehäuse aufweist, wobei die Verarbeitungseinheit so konfiguriert ist, dass sie bei der Bestimmung der Kraft den gemessenen Gasdruck ($F_{Gehäuse}$) berücksichtigt.

14. System nach einem beliebigen der vorstehenden Ansprüche 1 bis 13, wobei eine Wand des Behälters mit variablem Volumen durch eine Gehäusewand gebildet wird und die Wand eine perforierbare Einstichstelle (4) aufweist.

15. System nach einem beliebigen der vorstehenden Ansprüche 1 bis 14, das ferner eine Vorrichtung zum Reduzieren der Belastungen im Fluidkreislauf aufweist, wenn diese Belastungen einen vorbestimmten Schwellenwert überschreiten.

16. System nach einem beliebigen der vorstehenden Ansprüche 1 bis 15, das ferner einen Beschleunigungsmesser aufweist, wobei die Verarbeitungseinheit so konfiguriert ist, dass sie ausgehend von Messdaten des Beschleunigungsmessers bestimmt, ob sich der Patient in einer bestimmten Lage befindet.

17. System nach einem beliebigen der vorstehenden Ansprüche 1 bis 16, wobei die Druckmessvorrichtung so konfiguriert ist, dass sie beim Einstellen des Volumens des Behälters den Fluiddruck misst und die Übereinstimmung zwischen dem gemessenen Wert und einem erwarteten Wert überprüft.

18. System nach einem beliebigen der vorstehenden Ansprüche 1 bis 17, das aus einem künstlichen Harnschließmuskel besteht.

19. System nach einem der Ansprüche 1 bis 18, wobei die Verarbeitungseinheit so konfiguriert ist, dass sie einen relativen Fluiddruck (P2) im Fluidkreislauf berechnet, wobei der relative Druck gleich der Differenz zwischen dem im Fluidkreislauf bestimmten Fluiddruck (P1) und dem auf den Patienten einwirkenden atmosphärischen Druck (Patm) ist.

20. System nach Anspruch 19, das ferner eine Vorrichtung umfasst, die außerhalb des Körpers des Patienten platziert werden soll, und einen barometrischen Sensor, der geeignet ist, den auf den Patienten einwirkenden atmosphärischen Druck zu messen, wobei die Vorrichtung in der Lage ist, zur Berechnung des relativen Drucks des Fluids im Fluidkreislauf die Druckmessung an die Verarbeitungseinheit zu übermitteln.

21. System nach Anspruch 19, wobei die Verarbeitungseinheit so konfiguriert ist, dass sie den auf den Patienten einwirkenden atmosphärischen Druck ausgehend vom Druck im Fluidkreislauf und einem bekannten relativen Druck in einer gegebenen Betätigungskonfiguration der Manschette bestimmt.

**Claims**

1. An occlusion system implantable in a human or animal body, comprising:

    - a fluidic circuit comprising:

        • an inflatable occlusive sleeve (3) containing a variable volume of a fluid, intended to surround at least one portion of a natural conduit to be occluded,
        • a reservoir (5) with variable volume filled with a fluid, said reservoir comprising a fixed portion and a movable portion,
        • a fluidic connection (2) between the reservoir (5) and the occlusive sleeve (3),

    - an actuator mechanically coupled with the movable portion of the reservoir so as to linearly displace said movable portion relatively to the fixed portion for adjusting the volume of the reservoir,

    the actuator and the reservoir with variable volume being laid out in a sealed casing (1) containing a gas,
    said system being **characterized in that** it further comprises:

        - a sensor (21, 22) laid out in the casing (1), mechanically bound to the actuator and/or to the movable portion of the reservoir, laid out so as to measure a traction and/or compressive force in the direction of displacement of the movable portion of the reservoir, said measured force resulting at least from:

            • the force ($F_{occl}$) exerted on the movable portion of the reservoir with variable volume related to the pressure in the fluidic circuit, and
            • the force ($F_{casing}$) exerted on the movable portion of the reservoir with a variable volume related to the gas pressure in the casing,

        - a device for measuring the fluid pressure (P1) in the fluidic circuit comprising a processing unit configured so as to determine said fluid pressure (P1) from a calculation taking into account at least the force measured by said sensor (21, 22), the effective pressure surface area ($S_{eff}$) of the movable portion of the reservoir, and the force ($F_{casing}$) exerted on the movable portion of the reservoir with a variable volume related to the gas pressure in the casing.

2. The system according to claim 1, further comprising a device for controlling the fluid pressure in the fluidic circuit by the volume of the reservoir, comprising:

        • a memory in which is recorded a relationship between the pressure in the fluidic circuit and the volume of said reservoir,
        • a processing unit configured for:

            - receiving a set fluid pressure value in the fluidic circuit,
            - from the relationship recorded in the memory between the pressure in the reservoir and the volume of the reservoir, determining the volume of the reservoir with which it is possible to attain the set pressure value,
            - if necessary, controlling the actuator in order to displace the movable portion of the reservoir in the position defining said determined volume,

        • a calibration unit configured for:

            (a) when the patient is in a determined situation, controlling the actuator in order to displace the movable portion of the reservoir in a plurality of determined positions, each position defining a determined volume of the reservoir,

(b) for each of set positions:

- measuring the fluid pressure (P1) in the fluidic circuit with said device for measuring the fluid pressure in the fluidic circuit,
- updating the memory by recording said measured fluid pressure in the fluidic circuit for the respective volume of the reservoir.

3. The system according to one of claims 1 or 2, wherein said sensor is able to measure traction forces and compressive forces in the direction of displacement of the movable portion of the reservoir.

4. The system according to one of claims 1 or 2, wherein said sensor is able to only measure compressive forces in the direction of displacement of the movable portion of the reservoir, said system further comprising a pre-stress device laid out so as to exert a determined compressive pre-stress on said sensor.

5. The system according to claim 4, wherein the processing unit is configured so as to take into account said pre-stress for determining the fluid pressure (P1) in the fluidic circuit.

6. The system according to one of claims 4 or 5, wherein said pre-stress device comprises at least one compressive spring, a traction spring and/or an elastomeric pad.

7. The system according to one of claims 1 to 6, wherein the movable portion of the reservoir with variable volume comprises a drive system (10, 17) coupled with a movable wall (6) and deformable bellows extending and being compressed depending on the position of said movable wall.

8. The system according to claim 7, wherein the processing unit is configured for taking into account the stiffness of said bellows for determining the fluid pressure (P1) in the fluidic circuit.

9. The system according to one of claims 7 or 8, wherein:

- the drive system comprises a screw (17) secured to the movable wall (6) and a nut (10) coupled with the screw (17) and movable in rotation on a dual effect thrust ball bearing (16) around the axis of the screw under the effect of a driving action by the actuator, the nut (10) being coupled with the screw (17) so that a rotation of the nut (10) drives the screw (17) only in translation in the displacement direction of the movable portion (6),
- the sensor (22) is laid out in said thrust ball bearing (16) so as to measure at least one traction force and one compression force in the displacement direction of the movable portion of the reservoir.

10. The system according to one of claims 1 to 6, wherein the movable portion of the reservoir with variable volume comprises a drive system (17) coupled with a rolling membrane.

11. The system according to one of claims 1 to 6, wherein the reservoir with variable volume comprises a cylinder forming the fixed portion of the reservoir and a piston sliding in said cylinder, forming the movable portion of the reservoir.

12. The system according to one of claims 1 to 11, wherein the actuator is selected from piezoelectric actuators, electromagnetic actuators, electro-active polymers and shape memory alloys.

13. The system according to one of claims 1 to 12, further comprising a gas pressure sensor laid out in the casing (1) for measuring the gas pressure in the casing, the processing unit being configured for taking into account said measured gas pressure in the determination of the force ($F_{casing}$).

14. The system according to one of claims 1 to 13, wherein a wall of the reservoir with a variable volume is formed by a wall of the casing, said wall comprising a puncture port (4) which may be perforated.

15. The system according to one of claims 1 to 14 further comprising a device for reducing stresses in the fluidic circuit when said stresses exceed a determined threshold.

16. The system according to one of claims 1 to 15, further comprising an accelerometer, the processing unit being configured for, from measurement data of the accelerometer, determining whether the patient is in a determined situation.

17. The system according to one of claims 1 to 16, wherein the device for measuring the pressure is configured for measuring the fluid pressure during adjustment of the volume of the reservoir and for checking the match between said measured value and an expected value.

18. The system according to one of claims 1 to 17, consisting in an artificial urinary sphincter.

19. The system according to one of claims 1 to 18, wherein the treatment unit is configured for calculating a relative fluid pressure (P2) in the fluidic circuit, said relative pressure being equal to the difference between the fluid pressure (P1) determined in the fluidic circuit, and the atmospheric pressure (Patm) exerted on the patient.

20. The system according to claim 19, further comprising a device intended to be placed outside the body of the patient and comprising a barometric sensor adapted for measuring the atmospheric pressure exerted on the patient, said device being capable of communicating said pressure measurement to the treatment unit for calculating said relative pressure of the fluid in the fluidic circuit.

21. The system according to claim 19, wherein the treatment unit is configured for determining the atmospheric pressure exerted on the patient, from the pressure in the fluid circuit and from a known relative pressure in a given actuation of the sleeve.

**FIGURE 1**

**FIGURE 2**

**FIGURE 3**

**FIGURE 4**

**FIGURE 5**

**FIGURE 6**

**FIGURE 7**

**FIGURE 8**

**FIGURE 9**

**FIGURE 10**

**FIGURE 11**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1584303 A
- US 8585580 B
- US 4222377 A
- CA 1248303
- US 4408597 A
- US 4581018 A

**Littérature non-brevet citée dans la description**

- **LAMRAOUI, H ; BONVILAIN, A ; ROBAIN, G ; COMBRISSON, H ; BASROUR, S ; MOREAU-GAUDRY, A ; CINQUIN, P ; MOZER, P.** Development of a Novel Artificial Urinary Sphincter: A Versatile Automated Device. *IEEE - ASME Transactions on Mechatronics,* 2010, vol. 15, 916-924